(19) Europäisches Patentamt
European Patent Office
Office européen des brevets

(11) Veröffentlichungsnummer: 0 000 155
A1

(12) **EUROPÄISCHE PATENTANMELDUNG**

(21) Anmeldenummer: 78100171.4

(22) Anmeldetag: 15.06.78

(51) Int. Cl.[2]: C 07 C 143/30, C 07 B 13/06

(30) Priorität: 22.06.77 DE 2728070

(43) Veröffentlichungstag der Anmeldung: 10.01.79 Patentblatt 79/1

(84) Benannte Vertragsstaaten: BE CH DE FR GB NL

(71) Anmelder: Bayer Aktiengesellschaft,
Zentralbereich Patente,Marken und Lizenzen Bayerwerk,
D-5090 Leverkusen 1 (DE)

(72) Erfinder: Alles, Hans-Ulrich, Dr.,
Reiner-Hütten-Strasse 15,
D-5068 Odenthal (DE)

(72) Erfinder: Schulz, Nikolaus, Dr.,
Lessingstrasse 66,
D-5000 Köln 40 (DE)

(72) Erfinder: Wunderlich, Hermann, Dr.,
Arndtstrasse 8,
D-5068 Odenthal (DE)

(72) Erfinder: Jakob, Rolf,
Am alten Hof 12,
D-5068 Odenthal (DE)

(54) **Kontinuierliche Herstellung von Naphthalin-1-sulfonsäure und -1,5-disulfonsäure.**

(57) Die Erfindung betrifft ein Verfahren zur Herstellung von Naphthalinsulfonsäuren durch Umsetzung von Naphthalin mit Schwefeltrioxid in Schwefelsäure und bei niederen Temperaturen in einer Schneckenmaschine.

EP 0 000 155 A1

BAYER AKTIENGESELLSCHAFT
Zentralbereich
Patente, Marken und Lizenzen

5090 Leverkusen, Bayerwerk
Mn/AB

# Kontinuierliche Herstellung von Naphthalin-1-sulfonsäure und -1,5-disulfonsäure

Die Erfindung betrifft ein Verfahren zur Herstellung von Naphthalin-1-sulfonsäure und -1,5-disulfonsäure durch Sulfonierung von Naphthalin.

Es ist bekannt, Naphthalin-1-sulfonsäure durch Sulfonieren von Naphthalin mit 96 %iger Schwefelsäure bei 20 bis 50°C und Naphthalin-1,5-disulfonsäure durch Sulfonieren von Naphthalin mit 20 %igem Oleum bei 20 bis 55°C herzustellen (Ullmanns Enzyclopädie der technischen Chemie, Band 12, (1960), Seiten 593 bis 595).

Es wurde ein Verfahren zur Herstellung von Naphthalinsulfonsäuren der Formel

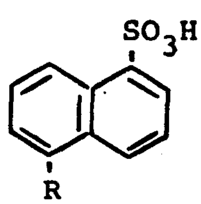

(I)

worin

R für Wasserstoff oder die $-SO_3H$-Gruppe steht,

durch Umsetzung von Naphthalin mit Schwefeltrioxid in Schwefelsäure und bei niederen Temperaturen gefunden, bei dem man die Umsetzung in einer Schneckenmaschine ausführt.

Schneckenmaschinen, wie sie für das erfindungsgemäße Verfahren verwendet werden können, sind bekannt (Herrmann, Schneckenmaschinen in der Verfahrenstechnik, Springer Verlag (1972)).

Eine Schneckenmaschine ist eine Vorrichtung, enthaltend eine oder mehrere Schnecken und ein die äußere Kontur der Wellen umschließendes Gehäuse. Schnecken bestehen aus drehenden Wellen mit außen in einer oder mehreren Schraubenlinien angebrachten Stegen. Schneckenmaschinen dienen zum Fördern, Mischen, Plastifizieren und/oder Verdichten von Stoffen.

Für das erfindungsgemäße Verfahren werden vorzugsweise Schneckenmaschinen eingesetzt, die eine gute Mischwirkung quer zur Förderrichtung und gleichzeitig ein enges Verweilzeitspektrum aufweisen. Besonders bevorzugt verwendet man für das erfindungsgemäße Verfahren einen einwelligen Schneckenkneter, dessen Schneckenwelle zusätzlich zur Rotation eine axial oscillierende Bewegung ausführt oder eine selbstreinigende gleichsinnig-drehende Doppelwellenschneckenmaschine, die zusätzlich zu den ineinandergreifenden Schneckengewinden noch sich ebenfalls gegenseitig abreinigende Knetelemente mit gegenüber den Schneckengewinden verstärkter Mischwirkung enthält.

Nach dem erfindungsgemäßen Verfahren können je Mol Naphthalin 1,0 bis 5,0 Mol Schwefeltrioxid verwendet werden. Bevorzugt kann man zur Herstellung von Naphthalin-1-sulfonsäure 1,0 bis 1,2 insbesondere 1,0 bis 1,1 Mol, Schwefeltrioxid und zur Herstellung von Naphthalin-1,5-disulfonsäure 2,0 bis 2,4, insbesondere 2,0 bis 2,1 Mol, Schwefeltrioxid verwenden.

Die Schwefelsäure kann in einer Menge von 0,4 bis 5,0 Mol $H_2SO_4$ je Mol Naphthalin verwendet werden. Bevorzugt kann man zur Herstellung von Naphthalin-1-sulfonsäure 0,5 bis 2,0 Mol, insbesondere 0,5 bis 0,9 Mol, $H_2SO_4$ je Mol Naphthalin und für die Herstellung von Naphthalin-1,5-disulfonsäure 0,9 bis 3,0 Mol, insbesondere 0,9 bis 1,5 Mol $H_2SO_4$, je Mol Naphthalin einsetzen.

Schwefelsäure kann für das erfindungsgemäße Verfahren beispielsweise als konzentrierte Schwefelsäure (Monohydrat) oder im Gemisch mit Schwefeltrioxid (als Oleum) eingesetzt werden.

Es ist auch möglich, das Schwefeltrioxid flüssig unverdünnt einzusetzen.

Bevorzugt wird Schwefeltrioxid in Form von Oleum eingesetzt, wobei der Gehalt an Schwefeltrioxid zwischen 20 und 65 Gew.-% liegen kann, bevorzugt wird das übliche etwa 65 %ige Oleum verwendet.

Im allgemeinen wird das erfindungsgemäße Verfahren im Temperaturbereich zwischen -10 und +25°C durchgeführt, bevorzugt im Temperaturbereich zwischen -5° und +15°C. Dabei ist es im allgemeinen vorteilhaft, bei Temperaturen über +5°C zur Herstellung von Naphthalin-1,5-disulfonsäure Schwefeltrioxid nur in einer Menge von 2,0 bis 2,5 Mol, insbesondere 2,0 bis 2,1 Mol, je Mol Naphthalin einzusetzen, während bei Temperaturen unterhalb +5°C auch größere Schwefeltrioxidmengen verwendet werden können. Ein derartiger Einsatz höherer Schwefeltrioxidmengen kann für eine Weiterverarbeitung der erhaltenen Naphthalin-1,5-disulfonsäure in Abwesenheit von Wasser vorteilhaft und erwünscht sein.

Im allgemeinen kann das erfindungsgemäße Verfahren wie folgt durchgeführt werden:

Um bei der exothermen Sulfonierungsreaktion die niedrige Reaktionstemperatur aufrecht erhalten zu können, werden vorteilhafterweise Schneckenmaschinen verwendet, die mit entsprechenden Kühlvorrichtungen ausgestattet sind.

Naphthalin kann in fester Form z.B. über eine Dosierschnecke oder in flüssiger Form über eine entsprechende Meßeinrichtung, z.B. eine Dosierpumpe, der Schneckenmaschine zugeführt werden. Es kann vorteilhaft sein, flüssiges Naphthalin vor der Zuführung zur Schneckenmaschine zu kühlen und kristallisieren zu lassen, z.B. in einer Kristallisierschnecke.

Um das Naphthalin auf die gewählte Reaktionstemperatur zu bringen, stattet man vorteilhafterweise die erste Zone der Schneckenmaschine mit einer Kühlung aus, um das Naphthalin auf oder unter die gewählte Reaktionstemperatur zu kühlen.

Nach Eingabe und gegebenenfalls Abkühlung des Naphthalins erfolgt die Zugabe der gewählten Menge Schwefelsäure an einer oder mehreren Stellen in Förderrichtung der Schneckenmaschine, wobei Schwefelsäure und Naphthalin gemischt werden. Wegen der bei der Reaktion auftretenden Wärmetönung kann es, wie bereits ausgeführt, vorteilhaft sein, die Zugabe der Schwefelsäure auf mehrere, in Förderrichtung hintereinander liegende Abschnitte der Schneckenmaschine zu verteilen und andererseits nach Zugabe der Schwefelsäure eine weitere Kühlstrecke vorzusehen.

Anschließend wird im folgenden Abschnitt der Schneckenmaschine Schwefeltrioxid in der gewählten Menge eindosiert; auch diese Eindosierung kann auf einmal oder an mehreren, in Förderrichtung hintereinander liegenden Stellen erfolgen.

Selbstverständlich kann bei der Verwendung von Oleum bereits zusammen mit Schwefelsäure Schwefeltrioxid in dem für die Zudosierung von Schwefelsäure vorgesehenen Abschnitt der Schneckenmaschine und/oder in dem für die Zudosierung von Schwefeltrioxid vorgesehenen Abschnitt der Schneckenmaschine noch Schwefelsäure zudosiert werden.

Es kann weiterhin vorteilhaft sein, das erhaltene Sulfiergemisch in der Schneckenmaschine noch eine nachgeschaltete, ebenfalls gekühlte Nachreaktionszone durchlaufen zu lassen.

Am Ausgang der Schneckenmaschine wird das Reaktionsgemisch je nach der verwendeten Menge Schwefelsäure als dickflüssige, pastenförmige Masse erhalten.

Zur Aufarbeitung kann das Sulfiergemisch anschließend auf Wasser oder Eis in bekannter Weise ausgetragen und die Naphthalin-1-sulfonsäure bzw. die Naphthalin-1,5-disulfonsäure in bekannter Weise z.B. durch Kristallisation in Form der Hydrate oder nach Zusatz von Natronlauge als Natriumsalze abgeschieden und anschließend isoliert werden (F.I.A.T. Final Report 1016 (1947). Das Sulfiergemisch kann auch ohne Verdünnung mit Wasser oder Eis für Folgereaktionen verwendet werden.

In einer bevorzugten Ausführungsform des erfindungsgemäßen Verfahrens wird die Sulfierung in einem Verdünnungsmittel durchgeführt, wobei als Verdünnungsmittel vorzugsweise das Umsetzungsprodukt des Verfahrens eingesetzt wird.

In dieser besonderen Ausführungsform des erfindungsgemäßen Verfahrens wird ein Teil des Reaktionsgemisches vom Ausgang der Schneckenmaschine wieder zum Eingang zurückgeführt und zusammen mit neuem Ausgangsmaterial erneut eingegeben; ein Teil des Umsetzungsproduktes wird also im Kreis geführt.

Im allgemeinen werden bis zu 90%, bevorzugt 40 bis 80, insbesondere 50 bis 75 % des gesamten Reaktionsgemisches so im Kreis geführt, während nur der verbleibende Rest am Ausgang der Knetmaschine abgezogen und aufgearbeitet wird.

Die Rückführung des im Kreis geführten Reaktionsgemisches kann in an sich bekannter Weise, z.B. durch eine Förderschnecke erfolgen.

Diese besondere Ausführungsform des erfindungsgemäßen Verfahrens hat den Vorteil, daß die einzusetzende Schwefelsäuremenge besonders niedrig gehalten werden kann, sie beträgt dann im allgemeinen zwischen 0,5 bis 3,0, bevorzugt 0,7 bis 1,5 Mol, je Mol Naphthalin.

Nach dem bekannten Verfahren werden Sulfonierungen in Rührkesseln durchgeführt, in denen jedoch wegen des ungünstigen Verhältnisses von Rauminhalt zu Wandfläche die Abführung großer Wärmemengen in kurzer Zeit erhebliche Schwierigkeiten bereitet. Entweder muß man einen der Reaktionspartner sehr langsam zugeben, so daß lange Reaktionszeiten notwendig sind, oder in Verdünnung mit niedrigen Konzentrationen der Reaktionspartner arbeiten. Als Verdünnungsmittel sind z.B. Schwefelsäure, flüssiges Schwefeldioxid und Chlorkohlenwasserstoffe bekannt. Wegen der niedrigen Reaktionstemperatur liegen Naphthalin und die Naphthalinsulfonsäuren in fester Form vor, so daß eine gewisse Menge Lösungsmittel, im allgemeinen Schwefelsäure, notwendig ist; beim Sulfonieren mit Schwefelsäure ist dieser Überschuß gleichzeitig notwendig, um das entstandene Reaktionswasser aufzunehmen und eine zu starke Verdünnung des Sulfonierungsmittels Schwefelsäure zu verhindern. Da bei niedrigem Lösungsmittelzusatz die Viskosität des Reaktionsgemisches leicht zu hoch wird, so daß eine ausreichende Durchmischung und damit schnelle Abführung

der Reaktionswärme an die gekühlten Wände nicht mehr gewährleistet ist, kann die Lösungsmittelmenge auch nicht zu niedrig bemessen sein (Literaturzitat).
Der Einsatz von Lösungsmitteln oder überschüssiger Schwefelsäure schafft weitere Probleme der Rückgewinnung oder des Verlustes und des Umweltschutzes.

Demgegenüber werden vorteilhafterweise nach dem erfindungsgemäßen Verfahren wesentlich geringere Mengen Schwefelsäure als Verdünnungs- bzw. Lösungsmittel verwendet, so daß sich mit dem geringeren Anfall an Schwefelsäure auch entsprechend die Probleme des Umweltschutzes vermindern.

Ferner ergeben sich durch die besseren Möglichkeiten der Temperaturregelung geringere Mengen unerwünschter Nebenprodukte. Weiterhin ergeben sich aus der besseren Temperaturführung Vorteile bei der Dimensionierung der Kühlaggregate und Nutzung der zugeführten Kühlenergie.

Naphthalin-1-sulfonsäure und -1,5-disulfonsäure sind bekannte technische Zwischenprodukte.

Beispiel 1

In eine zweiwellige selbstreinigende Gleichdrallschnecke von 1200 mm Länge, 32 mm Wellendurchmesser und 500 ml freiem Volumen mit einer Kombination von Gewinde und Knetelementen und mit solegekühltem Gehäuse werden bei einer Drehzahl von 100 Umdrehungen/min. je Stunde 1,0 kg (7,8 Mol) Naphthalin am Schneckeneinzug eindosiert. In Förderrichung 10 cm hinter dem Einzug werden pro Stunde 1,22 kg (12,5 Mol) Schwefelsäure und 20 cm hinter dem Einzug pro Stunde 4,06 kg 65 %iges Oleum (65 Gew.-% (33 Mol) Schwefeltrioxid und 35 Gew.-% (14,5 Mol) Schwefelsäure) eingepumpt. Die Temperaturen an den Dosierstellen des Schwefeltrioxids werden so geführt, daß eine Temperatur von +5°C nicht überschritten wird.

Es werden pro Stunde 6,28 kg eines Gemisches aus ca. 37 % Naphthalinsulfonsäure, ca. 42 % Schwefelsäure und ca. 21 % Schwefeltrioxid erhalten.

Die Sulfonsäuren setzen sich wie folgt zusammen:

73 Mol-% Naphthalin-1,5-disulfonsäure
9 Mol-% Naphthalin-1,6-disulfonsäure
5 Mol-% andere Naphthalin-disulfonsäuren
2 Mol-% Naphthalin-monosulfonsäuren
11 Mol-% Naphthalin-trisulfonsäuren.

Beispiel 2

In die in Beispiel 1 beschriebene Schneckenmaschine werden je Stunde 0,7 kg (5,5 Mol) Naphthalin, 0,9 kg (9,2 Mol) Schwefelsäure und 2,9 kg 65 %iges Oleum (23,5 Mol Schwefeltrioxid, 10,5 Mol Schwefelsäure) bei 5°C eindosiert.

Das Reaktionsgemisch wird über eine zweite auf 5°C gekühlte 2-wellige Gleichdrallschnecke 800 mm Länge, 32 mm Wellendurchmesser ohne Knetelemente an den Eingang der Reaktionsschneckenmaschine zurückgeführt.

Nach Füllung des gesamten Apparatevolumens werden hinter der Rückführungsschnecke 4,5 kg des Reaktionsgemisches je Stunde entnommen.

Das Sulfonsäure-Gemisch hat folgende Zusammensetzung:

76 Mol-% Naphthalin-1,5-disulfonsäure
12 Mol-% Naphthalin-1,6-disulfonsäure
6 Mol-% andere Naphthalin-disulfonsäuren
5 Mol-% Naphthalin-trisulfonsäuren.

Beispiel 3

In die in Beispiel 2 beschriebene Apparatur werden bei einer Reaktionstemperatur von 15°C je Stunde 1,2 kg (9,4 Mol) Napthalin, 0,16 kg (1,64 Mol) Schwefelsäure und 1,21 kg 65 %iges Oleum (9,9 Mol Schwefeltrioxid, 4,3 Mol Schwefelsäure) eindosiert.

An einer zusätzlichen Dosierstelle 40 cm hinter dem Schneckeneinzug werden je Stunde weitere 1,21 kg 65 %iges Oleum eingespeist.

Nach Füllung der Apparatur werden je Stunde 3,78 kg Reaktionsgemisch, bestehend aus ca. 72,5 % Naphthalinsulfonsäuren, ca. 27 % Schwefelsäure und ca. 0,5 % Schwefeltrioxid, entnommen.

Die Sulfonsäuren setzen sich wie folgt zusammen:

71 Mol-% Naphthalin-1,5-disulfonsäure
15 Mol-% Naphthalin-1,6-disulfonsäure
8 Mol-% andere Naphthalin-disulfonsäuren
5 Mol-% Naphthalin-trisulfonsäuren.

Das am Ende der Schneckenmaschine abgezogene Reaktionsgemisch wird wie folgt aufgearbeitet:
1 kg des Reaktionsgemisches wird in Wasser oder Wasser/Eis-Gemisch ausgetragen, so daß sich die Temperatur der entstehenden Lösung bei 80 bis 90°C hält. Die Wassermenge wird so gewählt, daß die entstehende Lösung ca. 40 % Schwefelsäure enthält. Die heiße Lösung wird langsam auf 20°C abgekühlt, das anfallende Kristallisat abgeschleudert und mit 50 %iger Schwefelsäure gewaschen.

Das gewaschene, gut abgeschleuderte Feuchtgut enthält ca. 95 % Naphthalin-1,5-disulfonsäure (als Tetrahydrat) und ca. 5 % Restfeuchte (als 50 %ige Schwefelsäure).

<u>Beispiel 4</u>

In die in Beispiel 3 beschriebene Apparatur werden je Stunde 1,2 kg (9,4 Mol) Naphthalin, 0,33 kg (3,4 Mol) Schwefelsäure und zwei mal je 0,605 kg 65 %iges Oleum (4,9 Mol Schwefeltrioxid, 2,1 Mol Schwefelsäure) eindosiert.
Dabei wird im Gegensatz zu Beispiel 3 in der Reaktionsschnecke eine Temperatur von 0°C und in der Rückführungsschnecke von +10°C eingehalten.

Nach Füllung der Apparatur werden hinter der Rückführungsschnecke je Stunde 2,74 kg des Reaktionsgemisches, bestehend aus ca. 74 % Naphthalinsulfonsäuren, ca. 25,5 % Schwefelsäure und ca. 0,5 % Schwefeltrioxid aufgearbeitet.

Das Sulfonsäuregemisch hat folgende Zusammensetzung:

78 Mol-% Naphthalin-1-sulfonsäure
14 Mol-% Naphthalin-2-sulfonsäure
6 Mol-% Naphthalin-disulfonsäuren
2 Mol-% Naphthalin-disulfonsäuren

Patentansprüche

1. Verfahren zur Herstellung von Naphthalinsulfonsäuren der Formel

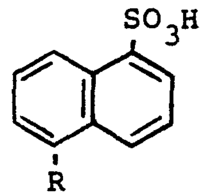

worin

R für Wasserstoff oder die $-SO_3H$-Gruppe steht,

durch Umsetzung von Naphthalin mit Schwefeltrioxid in Schwefelsäure und bei niederen Temperaturen, dadurch gekennzeichnet, daß man die Umsetzung in einer Schneckenmaschine ausführt.

2. Verfahren nach Anspruch 1, dadurch gekennzeichnet, daß man einen Teil des Reaktionsgemisches vom Ausgang der Schneckenmaschine wieder zum Eingang zurückführt und zusammen mit neuem Ausgangsmaterial eingibt.

Europäisches Patentamt

# EUROPÄISCHER RECHERCHENBERICHT

0000155

Nummer der Anmeldung

EP 78 10 0...

| EINSCHLÄGIGE DOKUMENTE | | | KLASSIFIKATION DER ANMELDUNG (Int.Cl.²) |
|---|---|---|---|
| Kategorie | Kennzeichnung des Dokuments mit Angabe, soweit erforderlich, der maßgeblichen Teile | betrifft Anspruch | |
| | DE - C - 571 124 (H.Th.BOEHME A.G.)<br>* Seite 1; Zeile 66 bis Seite 2, Zeile 38; Abbildungen * | 1,2 | C 07 C 143/30<br>C 07 B 13/06 |
| | DE - C - 577 220 (H.Th.BOEHME A.G.)<br>* Seite 1, Zeilen 4-21; Abbildungen * | 1,2 | |
| | US - A - 3 495 951 (H.TANAKA)<br>* Spalte 6, Zeile 74 bis Spalte 7, Zeile 4; Spalte 7, Zeilen 35-54; Abbildung 3 * | 1 | |

RECHERCHIERTE SACHGEBIETE (Int. Cl.²)

C 07 C 143/30
C 07 B 13/06

KATEGORIE DER GENANNTEN DOKUMENTE

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

X Der vorliegende Recherchenbericht wurde für alle Patentansprüche erstellt.

| Recherchenort | Abschlußdatum der Recherche | Prüfer |
|---|---|---|
| Den Haag | 15-09-1978 | B... |